(19) Europäisches Patentamt
European Patent Office
Office européen des brevets

(11) EP 1 459 109 B1

(12) EUROPEAN PATENT SPECIFICATION

(45) Date of publication and mention of the grant of the patent:
**19.08.2009 Bulletin 2009/34**

(21) Application number: **02806216.4**

(22) Date of filing: **26.12.2002**

(51) Int Cl.:
***G02B 3/10*** *(2006.01)* ***G02C 7/04*** *(2006.01)*
***G02C 7/06*** *(2006.01)* ***A61F 2/16*** *(2006.01)*

(86) International application number:
**PCT/US2002/041319**

(87) International publication number:
**WO 2003/058296 (17.07.2003 Gazette 2003/29)**

(54) **LIGHT ADJUSTABLE MULTIFOCAL LENSES**

LICHTEINSTELLBARE MULTIFOKALLINSEN

LENTILLES MULTIFOCALES REGLABLES, TRANSPARENTES

(84) Designated Contracting States:
**AT BE BG CH CY CZ DE DK EE ES FI FR GB GR IE IT LI LU MC NL PT SE SI SK TR**

(30) Priority: **28.12.2001 US 347028 P**
**22.01.2002 US 350551 P**
**24.12.2002 US 328859**

(43) Date of publication of application:
**22.09.2004 Bulletin 2004/39**

(73) Proprietor: **Calhoun Vision Inc.**
**Pasadena, CA 91107 (US)**

(72) Inventors:
- **SANDSTEDT, Christian, A.**
**Pasadena, CA 91106 (US)**
- **JETHMALANI, Jagdish, M.**
**San Diego, CA 92130 (US)**
- **CHANG, Shiao, H.**
**Arcadia, CA 91006 (US)**

(74) Representative: **Jackson, Richard Eric et al**
**Carpmaels & Ransford**
**43-45 Bloomsbury Square**
**London WC1A 2RA (GB)**

(56) References cited:
**WO-A-01/71411**
**WO-A-98/05272**
**FR-A1- 2 657 294**
**US-A- 5 066 301**
**US-A- 5 443 506**
**US-A- 5 728 155**
**US-A1- 4 787 903**
**US-A1- 5 997 140**

EP 1 459 109 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### TECHNICAL FIELD

The invention relates to optical elements, which can be modified post-manufacture such that different versions of the element will have different optical properties. In one embodiment, it relates to lenses, such as intraocular lenses, which can be converted into multifocal lenses post-fabrication.

### BACKGROUND OF THE INVENTION

Accommodation, as it relates to the human visual system, refers to the ability of a person to use their unassisted ocular structure to view objects at both near (e.g. reading) and far (e.g. driving) distances. The mechanism whereby humans accommodate is by contraction and relaxation of the cilliary body which inserts into the capsular bag surrounding the natural lens. Under the application of cilliary stress, the human lens will undergo a shape change effectively altering the radius of curvature of the lens. This action produces a concomitant change in the power of the lens. However, as people grow older the ability for them to accommodate reduces dramatically. This condition is known as presbyopia and currently affects more than 90 million people in the US. The most widely believed theory to explain the loss of accommodation was put forth by Helmholtz and states that as the patient ages, the crystalline lens of the human eye becomes progressively stiffer prohibiting deformation under the applied action of the cilliary body.

People who can see objects at a distance without the need for spectacle correction, but have lost the ability to see objects up close are usually prescribed a pair of reading glasses or magnifiers. For those patients who have required previous spectacle correction due to preexisting defocus and/or astigmatism the patient is prescribed a pair of bifocals, trifocals, variable, or progressive focus lenses that allow the person to have both near and distance vision. Compounding this condition is the risk of cataract development as the patient ages. In fact, cataract extraction followed by intraocular lens (IOL) implantation is the most commonly performed surgery in patients over 65 years old (reference).

To effectively treat both presbyopia and cataracts the patient can be implanted with a multifocal IOL. The general concepts and designs of multifocal IOLs have been described before in the ophthalmic and patent literature. The simplest design for a multifocal IOL is commonly referred to as the "bull's eye" configuration and comprises a small, central add zone (1.5 mm to 2.5 mm in diameter) that provides near vision ("Intraocular Lenses in Cataract and Refractive Surgery," D. T. Azar, et. al., W. B. Saunders Company (2001); "Intraocular Lenses: Basics and Clinical Applications," R. L. Stamper, A Sugar, and D. J. Ripkin, American Academy of Ophthalmology (1993). The power of the central add zone is typically between 3 to 4 diopters greater than the base power of the IOL, which translates to an effective add of 2.5 to 3.5 diopters for the entire ocular system. The portion of the lens outside the central add zone is referred to as the base power and is used for distance viewing. In theory, as the pupil constricts for near viewing, only that central add zone of the lens will have light from the image passing through it. However, under bright viewing conditions the pupil will also constrict leaving the patient 2 to 3 diopters myopic. This can be potentially problematic for a person who is driving in a direction with the sun shining straight at them, e.g. driving west around the time of sunset. To counteract this problem, an annular design with the central and peripheral portion of the lens designed for distance viewing and a paracentral ring (2.1 to 3.5 mm) for near vision. This design will maintain.distance viewing even if the pupil constricts (Intraocular Lenses in Cataract and Refractive Surgery, D. T. Azar, et. al., W. B. Saunders Company (2001); "Intraocular Lenses: Basics and Clinical Applications," R. L. Stamper, A Sugar, and D. J. Ripkin, American Academy of Ophthalmology (1993). The most widely adopted multifocal IOL currently sold in the US is described in U.S. Patent No. 5,225,858. This IOL is known as the Array lens and comprises five concentric, aspheric annular zones. Each zone is a multifocal element and thus pupil size should play little or no role in determining final image quality.

However, as with standard intraocular lenses the power and focal zones of the lenses must be estimated prior to implantation. Errors in estimating the needed power as well as shifting of the lens post-operatively due to wound healing often results in less than optimal vision. The latter effect is particularly problematic for the case of the bull's eye lens if a transverse (perpendicular to the visual axis) shift of the IOL occurred during healing. This would effectively move the add part off the visual axis of the eye resulting in the lost of desired multifocality. The Array and paracentral IOL designs can partly overcome the dislocation problem during wound healing although any IOL movement longitudinally (the direction along the visual axis), preexisting astigmatism, or astigmatism induced by the surgical procedure can not be compensated using these multifocal IOL designs. This results in the patient having to choose between additional surgery to replace or reposition the lens or to use additional corrective lenses.

A need exists for an intraocular lens which can be adjusted post-operatively *in vivo* to form a multifocal intraocular lens. This type of lens can be designed in-vivo to correct to an initial emmetropic (light from infinity forming a perfect focus on the retina) state and then the multifocality may be added during a second treatment. Such a lens would remove some of the guess work involved in presurgical power selection, overcome the wound healing response inherent to IOL implantation, allow the size of the add or subtract zone(s) to be customized to correspond to the patient's magnitude

and characteristics of dilation under different illumination conditions, and allow the corrected zones to be placed along the patient's visual axis. WO 01/71411 A relates to methods of implementing an optical element having a refraction modulating composition. The methods include using a wavefront sensor to provide an optical measurement of the optical element. WO 01/71411 A also relates to systems comprising an optical element having a refraction modulating composition and a wavefront sensor.

FR 2657294 relates to the manufacture of an artificial optical lens having any given power profile. Starting from an artificial optical lens having another power profile, this power profile is modified, by means of a physico-chemical treatment procedure, for example by means of photochemistry, leading to the desired power profile. Application, in particular, to contact lenses and to intra-occular implants.

WO 98/05272 A discloses prismatic intraocular lenses for restoring visual function to an eye having macular degeneration. The lenses each include a convex lens optic for receiving and focusing light rays, a prismatic wedge located posterior to the convex lens optic for receiving and directing light rays to a first portion of a retina of the eye, and means for in situ alteration of the optical characteristics of the intraocular lens to direct light rays to a second function portion of the retina.

## BRIEF SUMMARY OF THE INVENTION

The present invention provides a multifocal lens and a method of preparing a multifocal lens as claimed in the appendant claims.

Novel optical elements are provided whose properties can be adjusted post-manufacture to produce an optical element having different properties. Specifically, the invention relates to an intraocular lens that can be transformed into a multifocal lens after the lens has been implanted in the eye. In this manner, the intraocular and/or focal zones of the lens can be more precisely adjusted after the lens has been subjected to any post-operative migration, and can be based on input from the patient and standard refraction techniques rather than preoperative estimation.

The alteration of the optical element is accomplished through the use of a modifying composition ("MC") dispersed throughout the element. The MC is capable of polymerization when exposed to an external stimulus such as heat or light. The stimulus can be directed to one or more regions of the element causing polymerization of the MC only in the exposed regions. The polymerization of the MC causes changes in the optical properties of the element with exposed regions.

Upon polymerization, several changes occur within the optical element. The first change is the formation of a second polymer network comprising polymerized MC. The formation of this polymer network can cause changes in the optical properties of the element, namely the refractive index. In addition, when the MC polymerizes, a difference in the chemical potential between the polymerized and unpolymerized region is induced. This in turn causes the unpolymerized MC to diffuse within the element, thermodynamic equilibrium of the optical element is reestablished. If the optical element possesses sufficient elasticity, this migration of MC can cause swelling of the element in the area exposed to the stimulus. This, in turn, changes the shape of the element, causing changes in the optical properties. Depending upon the nature of the optical element, the MC incorporated into the element, the duration, and the spatial intensity profile of the stimulus either or both of these two changes can occur.

One key aspect of the present invention is that the optical elements are self-contained in that once fabricated, no material is either added or removed from the lens to obtain the desired optical properties.

It has been found that by exposing different regions of the optical element to varying degrees or in a predetermined pattern of external stimulus, it is possible to vary the optical properties of the element in different regions. For example, it is possible through the use of various patterns, to create a central zone with one set of optical properties, surrounded by concentric rings of differing optical properties. In this way, a multifocal lens can be created. In another embodiment, customized bifocal, multifocal, etc. patterns can be written on the lens in one treatment followed by a second treatment to lock-in the unreacted modifying composition present throughout the entire lens. Alternately, multiple treatments of customized patterns can be written on the lens to provide patients with vision without the need for spectacles.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

The invention is directed to the multifocal lens that can be adjusted post-operatively in vivo or ex vivo after manufacture

according to claims 1-15 and to the method for ex vivo preparing a multifocal lens according to claims 16-19.

BRIEF DESCRIPTION OF THE DRAWINGS

For a more complete understanding of the present invention, reference is now made to the following descriptions taken in conjunction with the accompanying drawing, in which:

FIGURES 1A and 1B depict a cross-section of an intraocular lens and a micrograph, according to an embodiment of the invention.

FIGURES 2A and 2B depict a cross-section of a multifocal intraocular lens and a micrograph, according to an embodiment of the invention.

FIGURES 3A and 3B depict interference fringes for a lens, according to an embodiment of the invention.

FIGURES 4A, 4B, AND 4C depict an example of reversible multifocality for a lens, according to an embodiment of the invention.

FIGURE 5 is an example of a lens made according to embodiments of the invention.

DETAILED DESCRIPTION OF THE INVENTION

The optical elements of the present invention are capable of post-fabrication alteration of optical properties. The elements are self-contained and do not require the addition or removal of materials to change the optical properties. Instead, the optical properties are altered by exposing a portion or portions of the optical element to an external stimulus which induces polymerization of a MC within the element. The polymerization of the MC, in turn, causes the change in optical properties.

The optical element of the invention has dispersed within it a MC. This MC is capable of diffusion within the element; can be readily polymerized by exposure to a suitable external stimulus; and is compatible with the materials used to make the optical element.

The optical element is typically made of a first polymer matrix. Illustrative examples of a suitable first polymer matrix include: polyacrylates such as polyalkyl acrylates and polyhydroxyalkyl acrylates; polymethacrylates such as polymethyl methacrylate ("PMMA"), polyhydroxyethyl methacrylate ("PHEMA"), and polyhydroxypropyl methacrylate ("HPMA"); polyvinyls such as polystyrene and polyvinylpyrrolidone ("PNVP"); polysiloxanes such as polydimethylsiloxane; polyphosphazenes, and copolymers of thereof. U.S. Patent No. 4,260,725 and patents and references cited therein provide more specific examples of suitable polymers that may be used to form the first polymer matrix.

In preferred embodiments, where flexibility is desired, the first polymer matrix generally possesses a relatively low glass transition temperature ("$T_g$") such that the resulting IOL tends to exhibit fluid-like and/or elastomeric behavior, and is typically formed by cross-linking one or more polymeric starting materials wherein each polymeric starting material includes at least one cross-linkable group. In the case of an intraocular lens, the $T_g$ should be less than 25°C. This allows the lens to be folded, facilitating implantation. In cases where rigidity is desired, the $T_g$ should generally be greater than 25°C.

Illustrative examples of suitable cross-linkable groups include but are not limited to hydride, acetoxy, alkoxy, amino, anhydride, aryloxy, carboxy, enoxy, epoxy, halide, isocyano, olefinic, and oxine. In more preferred embodiments, such polymeric starting material includes terminal monomers (also referred to as endcaps) that are either the same or different from the one or more monomers that comprise the polymeric starting material but include at least one cross-linkable group. In other words, the terminal monomers begin and end the polymeric starting material and include at least one cross-linkable group as part of its structure. Although it is not necessary for the practice of the present invention, the mechanism for cross-linking the polymeric starting material preferably is different than the mechanism for the stimulus-induced polymerization of the components that comprise the refraction modulating composition. For example, if the refraction modulating composition is polymerized by photoinduced polymerization, then it is preferred that the polymeric starting materials have cross-linkable groups that are polymerized by any mechanism other than photoinduced polymerization.

An especially preferred class of polymeric starting materials for the formation of the first polymer matrix is polysiloxanes (also known as "silicones") endcapped with a terminal monomer which includes a cross-linkable group selected from the group consisting of acetoxy, amino, alkoxy, halide, hydroxy, and mercapto. Because silicone IOLs tend to be flexible and foldable, generally smaller incisions may be used during the IOL implantation procedure. An example of an especially preferred polymeric starting materials are vinyl endcapped dimethylsiloxane diphenylsiloxane copolymer, silicone resin, and silicone hydride crosslinker that are crosslinked via an addition polymerization by platinum catalyst to form the silicone matrix. Other such examples may be found in US 5,236,970, US 5,376,694, US 5,278,258, US 5,444,106, and others similar to the described formulations.

The MC that is used in fabricating IOLs is as described above except that it has the additional requirement of biocompatibility. The MC is capable of stimulus-induced polymerization and may be a single component or multiple

components so long as: (i) it is compatible with the formation of the first polymer matrix; (ii) it remains capable of stimulus-induced polymerization after the formation of the first polymer matrix; and (iii) it is freely diffusable within the first polymer matrix. In general, the same type of monomers that are used to form the first polymer matrix may be used as components of the refraction modulating composition. However, because of the requirement that the MC monomers must be diffusable within the first polymer matrix, the MC monomers generally tend to be smaller (i.e., have lower molecular weights) than the first polymer matrix. In addition to the one or more monomers, the MC may include other components such as initiators and sensitizers that facilitate the formation of the second polymer network.

In preferred embodiments, the stimulus-induced polymerization is photopolymerization. In other words, the one or more monomers that comprise the refraction modulating composition each preferably includes at least one group that is capable of photopolymerization. Illustrative examples of such photopolymerizable groups include but are not limited to acrylate, allyloxy, cinnamoyl, methacrylate, stibenyl, and vinyl. In more preferred embodiments, the refraction modulating composition includes a photoinitiator (any compound used to generate free radicals) either alone or in the presence of a sensitizer. Examples of suitable photoinitiators include acetophenones (e.g., substituted haloacetophenones, and diethoxyacetophenone); 2,4-dichloromethyl-1,3,5-trazines; benzoin methyl ether; and o-benzoyl oximino ketone. Examples of suitable sensitizers include p-(dialkylamino)aryl aldehyde; N-alkylindolylidene; and bis[p-(dialkylamino)benzylidene] ketone.

Because of the preference for flexible and foldable IOLs, an especially preferred class of MC monomers is polysiloxanes endcapped with a terminal siloxane moiety that includes a photopolymerizable group. An illustrative representation of such a monomer is:

$$X\text{-}Y\text{-}X^1$$

wherein Y is a siloxane which may be a monomer, a homopolymer or a copolymer formed from any number of siloxane units, and X and $X^1$ may be the same or different and are each independently a terminal siloxane moiety that includes a photopolymerizable group. An illustrative example of Y includes:

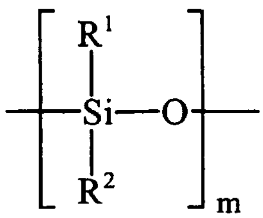

and

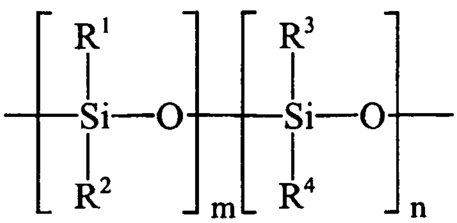

wherein: m and n are independently each an integer and

$R^1$, $R^2$, $R^3$, and $R^4$ are independently each hydrogen, alkyl (primary, secondary, tertiary, cyclo), aryl, or heteroaryl. In preferred embodiments, $R^1$, $R^2$, $R^3$, and $R^4$ are $C_1$-$C_{10}$ alkyl or phenyl. Because MC monomers with a relatively high aryl content have been found to produce larger changes in the refractive index of the inventive lens, it is generally preferred that at least one of $R^1$, $R^2$, $R^3$, and $R^4$ is an aryl, particularly phenyl. In more preferred embodiments, $R^1$, $R^2$, and $R^3$ are the same and are methyl, ethyl or propyl and $R^4$ is phenyl.

Illustrative examples of X and $X^1$ (or $X^1$ and X depending on how the MC polymer is depicted) are:

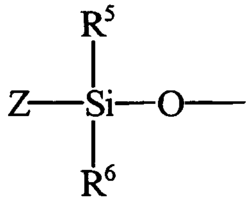

And

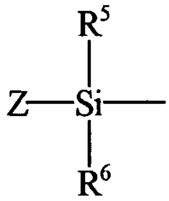

respectively wherein:

$R^5$ and $R^6$ are independently each hydrogen, alkyl, aryl, or heteroaryl; and

Z is a photopolymerizable group.

In preferred embodiments $R^5$ and $R^6$ are independently each $C_1$-$C_{10}$ alkyl or phenyl and Z is a photopolymerizable group that includes a moiety selected from the group consisting of acrylate, allyloxy, cinnamoyl, methacrylate, stibenyl, and vinyl. In more preferred embodiments, $R^5$ and $R^6$ are methyl, ethyl, or propyl and Z is a photopolymerizable group that includes an acrylate or methacrylate moiety.

In especially preferred embodiments, a MC monomer is of the following formula:

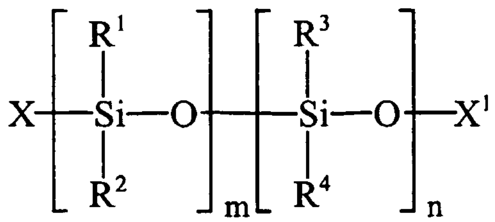

wherein X and $X^1$ are the same as $R^1$, $R^2$, $R^3$, and $R^4$ areas defined previously. Illustrative examples of such MC monomers include dimethylsiloxane-diphenylsiloxane copolymer endcapped with a vinyl dimethylsilane group; dimethylsiloxane-methylphenylsiloxane copolymer endcapped with a methacryloxypropyl dimethylsilane group; and dimethylsiloxane endcapped with a methacryloxypropyldimethylsilane group. Although any suitable method may be used, a ring-opening reaction of one or more cyclic siloxanes in the presence of triflic acid has been found to be a particularly efficient method of making one class of inventive MC monomers. Briefly, the method comprises contacting a cyclic siloxane with a compound of the formula:

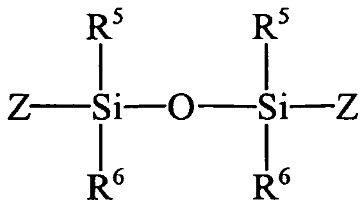

in the presence of triflic acid wherein $R^5$ and $R^6$, and Z are as defined previously. The cyclic siloxane may be a cyclic siloxane monomer, momopolymer, or copolymer. Alternatively, more than one cyclic siloxane may be used. For example, a cyclic dimethylsiloxane tetrameter and a cyclic methyl-phenylsiloxane trimer are contacted with bis-methacryloxypropyltetramethyldisiloxane in the presence of triflic acid to form a dimethylsiloxane methyl-phenylsiloxane copolymer that is endcapped with a methacryloxylpropyl-dimethylsilane group, an especially preferred MC monomer.

In addition to the silicone-based MCs described above, acrylate-based MC can also be used in the practice of the invention. The acrylate-based macromers of the invention have the general structure:

$$X\text{-}A_n\text{-}Q\text{-}A_n\text{-}X^1$$

or

$$X\text{-}A_n\text{-}A^1{}_m\text{-}Q\text{-}A^1{}_m\text{-}A_n\text{-}X^1$$

wherein Q is an acrylate moiety capable of acting as an initiator for Atom Transfer Radical Polymerization ("ATRP"), A and $A^1$ have the general structure:

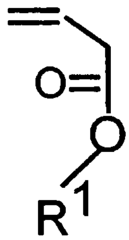

wherein $R^1$ is selected from the group comprising alkyls, halogenated alkyls, aryls and halogenated aryls and X and $X^1$ are groups containing photopolymerizable moieties and m and n are integers.

In one embodiment the acrylate based MC has the formula:

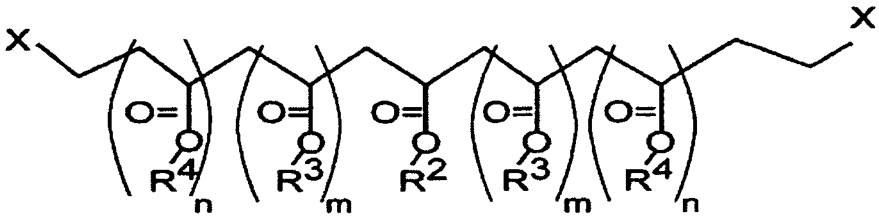

wherein $R^2$ is selected from the group comprising alkyls and halogenated alkyls $R^3$ and $R^4$ are different and are selected from the group consisting of alkyls, halogenated alkyls, aryls and halogenated aryls.

When the optical element is formed, it is then positioned in the area where it is to be used. For an intraocular lens, this means implantation into the eye using known procedures. Once the element is in place and is allowed to adjust to its environment, it is then possible to modify the optical properties of the element through exposure to an external stimulus.

The nature of the external stimulus can vary but it must be capable of reducing polymerization of the MC without adversely affecting the properties of the optical element. Typical external stimuli that can be used in practice of the invention include heat and light, with light preferred. In the case of intraocular lenses, ultraviolet or infrared radiation is preferred with ultraviolet light most preferred.

When the element is exposed to the external stimulus, the MC polymerization forms a second polymer matrix, interspersed with the first polymer matrix. When the polymerization is localized or when only a portion of the MC is polymerized, there is a difference in the chemical potential between the reacted and unreacted regions of the lens. The MC then migrates within the element to reestablish the thermodynamic equilibrium within the optical element.

The formation of the second polymer matrix and the re-distribution of the MC can each affect the optical properties of the element. For example, the formation of the second polymer matrix can cause changes in the refractive index of the element. The migration of the modifying compound can alter the overall shape of the element, further affecting the optical properties by changing the radii of curvatures of the optical element.

It is possible to localize the exposure of the optical element to the external stimulus in such a manner to create zones within the element with different optical properties. In one embodiment, it is possible to create an intraocular lens that can be transferred into a multifocal lens after implantation. This is accomplished by exposing the lens to different amounts of external stimulus to create zone(s) having different optical properties.

In the case of a multifocal intraocular lens, various methods can be used to create the lenses. In its simplest form, it can be of the bull's eye configuration comprising an add or subtract zone in the central 1 to 3 mm zone of the

lens and the resultant lens base power outside this zone. The lenses are divided into alternating zones. For example, separate zones would include outer and inner zones. A Fresnel lens is an example of alternating zones.

In the case of an intraocular lens, it is possible to form a lens, implant it, and then form different zones or regions in the lens having different optical properties. By exposing different areas of the lens to different magnitudes and spatial profiles of external stimuli, different optical zones can be created. For example, the lens body can be divided into central zone, inner and outer annular near zones, and annular far zones. In this embodiment, the central zone is circular and the peripheries of the annular zones are circular. The annular zones circumscribe the central zone and the zones are contiguous. The zones are concentric and coaxial with the lens body. When carried out ex vivo such a method forms part of the invention.

The zones are used in describing the vision correction power of the lens, and they are arbitrarily defined. Thus, the peripheries of the zones and the numbers of zones may be selected as desired.

The following examples are offered by way of example and are not intended to limit the scope of the invention in any manner.

EXAMPLE 1

A 6 mm diameter intraocular lens containing a silicone-based MC was prepared using standard molding techniques known to those skilled in the art. The lens had a first polymer matrix prepared from a silicone hydride crosslinked vinyl endcapped diphenylsiloxane dimethylsiloxane. The first polymer matrix comprised about 70 weight % of the lens. The lens also comprised about 30 weight % of a MC (methacrylate endcapped polydimethylsiloxane), 1 weight % (based on MC) of a photoinitiator (benzoin-tetrasiloxane-benzoin), and 0.04 weight % (based on MC) UV absorber. The lens had an initial nominal power of 30 diopters. The center of the lens was then irradiated with 365 nm light using an intensity pattern represented by the equation:

$$I = I_0 e^{-\frac{(r-r_c)^2}{2\sigma^2}} \qquad (1)$$

and an average intensity of 4.12 mW/cm$^2$ for 60 seconds. Three hours post-exposure, the lens had a +3.25 D change over the central 2.5 mm region of the lens, which is shown in FIGURE 1A. The interference fringes were taken at the preirradiation best focus position. The affected zone is easily observed in the central portion of the light adjustable lens (LAL) and is distinguished by the approximately 6 fringes (in double pass) of defocus in the central portion of the IOL. FIGURE 1B depicts a micrograph of FIGURE 1A.

In another embodiment, the first polymer matrix comprised about 75 weight % of the lens. The lens also comprised about 25 weight % of a MC (methacrylate endcapped methylphenylsiloxane dimethylsiloxane), 0.83 weight % (based on MC) of a photoinitiator (benzoin-L4-benzoin), and 0.04 weight % (based on MC) UV absorber. The lens had an initial nominal power of + 20.0 diopters. The lens was then irradiated with 365 nm ($\pm$ 5 nm) light using a spatial intensity profile described by the following equation:

$$I = I_0\left(0.65\frac{r^2}{r_{max}^2} + 0.35\right) \qquad (2)$$

The IOL was irradiated with an average intensity of 6 mW/cm$^2$ using three, 15 second exposures separated by 5 seconds. FIGURES 2A and 2B display the interference fringes (in double pass) of the lens before irradiation and 24 hours post irradiation. FIGURE 2A depicts the Fizeau interference fringe (in double pass) of a +20.0 D LAL at best focus preirradiation, the same LAL 24 hours after irradiation at the original best focus position. FIGURE 2B depicts the LAL of FIGURES 2A. The most striking feature between the two interferograms is the presence of a 3 mm reaction zone in the central portion of the lens, which is from the introduction of defocus. The change corresponds to a -0.70 diopters change in this central region.

These two examples illustrate that we can both add and subtract power from the central portion of the lens as well as control the effected zone size.

These two multifocal designs are similar to the bull's eye design described above. The difference between our design and those already presented in the literature and other patents is that we have the ability to affect the change

post-operatively after wound healing has occurred, customize the zone size to fit the patient's dilation conditions, add or subtract different amounts of power depending upon the recommendation of the patient or physician, and center the zone along the patient's visual axis once post-operative healing has finished.

EXAMPLE 2

One of the unique aspects of the above described lens is that we have the ability to first change the power of the IOL over the majority of its aperture and then reirradiate the lens over a small zone (0 to 3 mm) to create a bifocal lens as described in example 1. There are the advantages of first implanting the light adjustable lens in the patient, waiting the required healing time to let the eye refractively stabilize (typically two to four weeks), measuring the refraction of the patient to determine the necessary correction, if any, to bring the patient to emmetropia, irradiating the lens to change the power of the lens over the majority of the aperture, and then reirradiating a smaller zone in the lens (1.5-3 mm) along the patient's visual axis to provide the necessary multifocality for near and distance viewing.

As an example of this, a +20.0 D LAL was molded comprising 75 wt% of silicone matrix, 25 wt% of MC, 0.83 wt% PI, and 0.04 wt% UV absorber. The lens was initially irradiated using an average intensity of 10 mW/cm$^2$ using a spatial profile described by equation 2 above. The lens was dosed using seven 15 second exposures (5 seconds between each exposure). This treatment induced -1.32 diopters of change in the lens over a 5.5 region of the aperture. Twenty four hours post-irradiation, the lens was reirradiated in the central portion of the lens using the intensity profile represented by equation 1. The beam size was reduced to 3 mm in diameter, the average intensity of light was 6 mW/cm$^2$ and the dose was given in three 30 second doses. Twenty-four hours post irradiation; we observed a change of 1.94 diopters in this central region.

FIGURE 3A depicts Fizeau interference fringes (in double pass) of a +20.0 D LAL at best focus preirradiation. FIGURE 3B depicts the approximately 8 fringes (in double pass) of defocus introduced by the initial irradiation. This procedure introduced -1.32 diopters of change from the initial base power of +20.0 diopters. FIGURE 3C depicts the same LAL at the best focus position 24 hours after the initial irradiation. Note the presence of a new focus zone in the central part of the lens. This zone corresponds to +1.94 diopters of change.

EXAMPLE 3

In the past, the clinical use of bifocal or multifocal IOLs have met with some resistance by patients due to the loss of contrast sensitivity and glare that are inherent to this type of lens' designs. In the past, the only way for a physician to reverse the undesired affects of a previously implanted multifocal or bifocal IOL was to explant the IOL and reinsert it with a standard monofocal IOL. However, the light adjustable lens technology described in this disclosure and previous Calhoun Vision published works provides a means to reverse the multifocal properties of the LAL, effectively returning it to its monofocal condition. Such ability would have the oblivious advantage of reversal without surgical explantation.

As an example of this process, a +20.0 D LAL was molded comprising 75 wt% of silicone matrix, 25 wt% of MC, 0.83 wt% PI, and 0.04 wt% UV absorber. The preirradiation Fizeau interference fringes are shown in FIGURE 4A. This LAL was then irradiated using two successive, 30-second exposures of 6mW/cm$^2$. The spatial intensity profile of this initial irradiation is described by equation 2. As displayed in FIGURE 4B, -0.5 D of power were removed from the central optical zone of this lens. Twenty-four hours after this initial irradiation, the LAL was irradiated again using two successive, 30-second exposures of 3 mW/cm$^2$. The second irradiation effectively overlaid on top of the initial dose. The spatial intensity profile of this second irradiation is described by equation 1. This second irradiation added +0.5 D of power to the initially irradiated region, effectively removing the initial subtraction of power from the LAL and showing an example of multifocal reversibility in the Calhoun Vision LAL.

FIGURES 4A, 4B and 4C depict an example of reversible multifocality. FIGURE 4A depicts preirradiation Fizeau interference fringes of a +20.0 diopters LAL at best focus. FIGURE 4B Fizeau interference fringes at the preirradiation best focus 24 hours post initial irradiation. Note that -0.5 diopters of spherical power have been subtracted from the central portion of the LAL as noted by the fringes of defocus in the central portion of the LAL. FIGURE 4C depicts Fizeau interference fringes at the preirradiation best focus position two hours post the second irradiation showing the removal of the defocus fringes. This indicates that the LAL has been effectively brought back to its preirradiation power.

FIGURE 5 depicts an example of a lens 500 formed according to embodiments of the invention. The lens includes a plurality of different focal zones, 501, 502, 503, 504, 505, and 506. Note that the number of zones is by way of example only, as more or fewer zones could be used. The different zones are preferably concentric about a central zone 501. The different zones may have different radial widths, e.g. zone 504 has a smaller radial width than zone 503. Similarly, the different zones may have different areas, e.g. the area of zone 501 is smaller than the area of zone 503. Alternatively, some or all of the zones may have the same radial width and/or area as other zones. Each zone may have a different focal length or diopter than each of the other zones, e.g. zone 502 may be +1.0 diopter with respect to zone 501, and zone 503 may be +1.0 diopter with respect to zone 502, etc. Alternatively, some zones may have the same

power, while other zones have different powers. For example, zones 501, 503, and 505 may have the same power, while zones 502, 504, and 506 may be +1.0 diopter with respect to zone 501. As another example, zones 501, 503, and 505 may have the same power, while zone 502 may be +1.0 diopter with respect to zone 501, zone 504 may be +1.0 diopter with respect to zone 502, and zone 506 may be +1.0 diopter with respect to zone 504. Note that some zones may have a negative diopter with respect to other zones. Further note that the different zones may correct for nearsightedness, while other zones correct for farsightedness. The different zones may be in a pattern other than a "bulls-eye" patterns, e.g. a cylindrical pattern, which would be used to correct astigmatism. Any pattern zones may be formed into the lens. Lens 501 may be a eyeglass lens, a lens used in an optical system, or an intra-ocular lens, as long as the zones are arranged in alternating zones.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification.

## Claims

**1.** A multifocal lens comprising:

> a first portion of the lens has a first focal length;
> a second portion of the lens that includes a material that is optically reactive to an external stimulus and has a focal length that is adjusted to a second focal length by application of the stimulus;

wherein the first focal length is different from the second focal length, and wherein first and second portions are arranged as alternating zones.

**2.** The lens of claim 1, wherein the first focal length is greater than the second focal length.

**3.** The lens of claim 1, wherein the second focal length is greater than the first focal length.

**4.** The lens of claim 1, wherein the first portion has a substantially circular shape and is located at a center of the lens, and the second portion has a substantially annulus shape and is located around the first portion.

**5.** The lens of claim 1, wherein the second portion has a substantially circular shape and is located at a center of the lens, and the first portion has a substantially annulus shape and is located around the second portion.

**6.** The lens of claim 1, wherein the first portion of the lens includes a material that is optically reactive to an external stimulus.

**7.** The lens of claim 1, further comprising:

> a third portion of the lens that includes a material that is optically reactive to an external stimulus and has a focal length that is adjusted to a third focal length by application of the stimulus.

**8.** The lens of claim 7, wherein the third portion has a substantially annulus shape and is located around the first portion.

**9.** The lens of claim 7, wherein the third focal length is different from the first and second focal length.

**10.** The lens of claim 7, wherein the third focal length is the same as the second focal length.

**11.** The lens of claim 1 wherein said stimulus is light.

**12.** The lens of claim 11 wherein said stimulus is ultraviolet light.

**13.** The lens of claim 1 wherein said lens is an intraocular lens.

**14.** The lens of claim 1 wherein said lens is a contact lens.

**15.** The lens of claim 1 wherein said lens is a spectacle lens.

**16.** A method for preparing a multifocal lens ex vivo, comprising:

preparing a lens having a modifying composition (MC) dispersed therein, wherein the modifying composition is capable of stimulus-induced polymerization;
exposing a plurality of portions of the lens to an external stimulus that causes changes in the optical properties that change a focal length of each of the respective portions of the lens;
whereby each of the exposed portions of the lens has a respective focal length that is different from the focal length of an unexposed portion, and wherein said exposed portions and said unexposed portion are arranged as alternating zones.

**17.** The method of claim 16, further comprising: locking in the focal lengths of the exposed and unexposed portions.

**18.** The method of claim 16 wherein said external stimulus comprises ultraviolet light.

**19.** The method of claim 16 wherein said changes in optical properties are caused by polymerization of the MC in the regions exposed to the external stimulus.

**Patentansprüche**

**1.** Multifokallinse bzw. Mehrstärkenglas, die bzw. das umfasst:

einen ersten Abschnitt der Linse mit einer ersten Brennweite;
einen zweiten Abschnitt der Linse, der ein Material umfasst, das auf eine Anregung von außen optisch reagiert und eine Brennweite hat, die durch die Anwendung der Anregung auf eine zweite Brennweite eingestellt wird;

wobei die erste Brennweite sich von der zweiten Brennweite unterscheidet und wobei die ersten und zweiten Abschnitte als abwechselnde Zonen angeordnet sind.

**2.** Linse nach Anspruch 1, wobei erste Brennweite größer als die zweite Brennweite ist.

**3.** Linse nach Anspruch 1, wobei die zweite Brennweite größer als die erste Brennweite ist ist.

**4.** Linse nach Anspruch 1, wobei der erste Abschnitt eine im Wesentlichen kreisförmige Form hat und in einer Mitte der Linse angeordnet ist und der zweite Abschnitt eine im Wesentlichen ringförmige Form hat und um den ersten Abschnitt herum angeordnet ist.

**5.** Linse nach Anspruch 1, wobei der zweite Abschnitt im Wesentlichen eine kreisförmige Form hat und in einer Mitte der Linse angeordnet ist und der erste Abschnitt eine im Wesentlichen ringförmige Form hat und um den zweiten Abschnitt herum angeordnet ist.

**6.** Linse nach Anspruch 1, wobei der erste Abschnitt der Linse ein Material umfasst, das auf eine Anregung von außen optisch reagiert.

**7.** Linse nach Anspruch 1, die ferner umfasst:

einen dritten Abschnitt der Linse, der ein Material umfasst, das auf eine Anregung von außen optisch reagiert und eine Brennweite hat, die durch die Anwendung der Anregung auf eine dritte Brennweite eingestellt wird.

**8.** Linse nach Anspruch 7, wobei der dritte Abschnitt eine im Wesentlichen ringförmige Form hat und um den ersten Abschnitt herum angeordnet ist.

**9.** Linse nach Anspruch 7, wobei die dritte Brennweite sich von der ersten und der zweiten Brennweite unterscheidet.

**10.** Linse nach Anspruch 7, wobei die dritte Brennweite die gleiche wie die zweite Brennweite ist.

**11.** Linse nach Anspruch 1, wobei die Anregung Licht ist.

**12.** Linse nach Anspruch 11, wobei die Anregung Ultraviolettlicht ist.

**13.** Linse nach Anspruch 1, wobei die Linse eine Intraokularlinse ist.

**14.** Linse nach Anspruch 1, wobei die Linse eine Kontaktlinse ist.

**15.** Linse nach Anspruch 1, wobei die Linse ein Brillenglas ist.

**16.** Verfahren zum Herstellen einer Multifokallinse ex vivo bzw. außerhalb eines lebenden Organismus, das umfasst:

Herstellen einer Linse mit einer darin verteilten Modifikatorzusammensetzung (MC), wobei die Modifikatorzusammensetzung zu einer anregungsinduzierten Polymerisation fähig ist;
Aussetzen einer Vielzahl von Abschnitten der Linse einer Anregung von außen, die Änderungen in den optischen Eigenschaften bewirkt, welche eine Brennweite jedes der jeweiligen Abschnitte der Linse ändern;

wobei jeder der betroffenen bzw. belichteten Abschnitte eine jeweilige Brennweite hat, die sich von der Brennweite eines unbetroffenen Abschnitts unterscheidet, und wobei die betroffenen Abschnitte und die unbetroffenen Abschnitte als abwechselnde Zonen angeordnet sind.

**17.** Verfahren nach Anspruch 16, das ferner das Stabilhalten bzw. Sichern der Brennweiten der betroffenen und unbetroffenen Abschnitte umfasst.

**18.** Verfahren nach Anspruch 16, wobei die Anregung von außen Ultraviolettlicht umfasst.

**19.** Verfahren nach Anspruch 16, wobei die Änderungen der optischen Eigenschaften durch die Polymerisation der MC in den Bereichen bewirkt werden, die der Anregung von außen ausgesetzt werden.

## Revendications

**1.** Lentille progressive comprenant :

- une première partie de lentille ayant une première distance focale ;
- une seconde partie de lentille comportant un matériau capable de réagir sur le plan de l'optique à un stimulus externe et qui présente une distance focale qui est ajustée à une seconde distance focale par application du stimulus ;

dans laquelle la première distance focale est différente de la seconde distance focale, et dans laquelle les première et seconde parties sont disposées dans des zones en alternance.

**2.** Lentille selon la revendication 1, dans laquelle la première distance focale est supérieure à la seconde distance focale.

**3.** Lentille selon la revendication 1, dans laquelle la seconde distance focale est supérieure à la première distance focale.

**4.** Lentille selon la revendication 1, dans laquelle la première partie présente une forme sensiblement circulaire et est située au centre de la lentille, et la seconde partie présente une forme sensiblement annulaire et est située autour de la première partie.

**5.** Lentille selon la revendication 1, dans laquelle la seconde partie présente une forme sensiblement circulaire et est située au centre de la lentille, et la première partie présente une forme sensiblement annulaire et est située autour de la seconde partie.

**6.** Lentille selon la revendication 1, dans laquelle la première partie de la lentille comporte un matériau capable de réagir sur le plan de l'optique à un stimulus externe.

**7.** Lentille selon la revendication 1, comprenant en outre :

- une troisième partie de lentille comportant un matériau capable de réagir sur le plan de l'optique à un stimulus externe et qui présente une distance focale qui est ajustée à une troisième distance focale par application du stimulus.

**8.** Lentille selon la revendication 7, dans laquelle la troisième partie présente une forme sensiblement annulaire et est située autour de la première partie.

**9.** Lentille selon la revendication 7, dans laquelle la troisième distance focale est différente des première et seconde distances focales.

**10.** Lentille selon la revendication 7, dans laquelle la troisième distance focale est identique à la seconde distance focale.

**11.** Lentille selon la revendication 1, où ledit stimulus est la lumière.

**12.** Lentille selon la revendication 11, où ledit stimulus est une lumière ultraviolette.

**13.** Lentille selon la revendication 1, ladite lentille étant une lentille intraoculaire.

**14.** Lentille selon la revendication 1, ladite lentille étant une lentille de contact.

**15.** Lentille selon la revendication 1, ladite lentille étant un verre de lunette.

**16.** Procédé de fabrication d'une lentille progressive *ex vivo,* comprenant :

- la préparation d'une lentille renfermant, dispersée dans celle-ci, une composition modificatrice (CM) capable de faire l'objet d'une polymérisation induite par un stimulus ;
- l'exposition d'une pluralité de parties de la lentille à un stimulus externe qui provoque le changement des propriétés optiques modifiant la distance focale de chacune des parties respectives de la lentille ;

où chacune desdites parties exposées de la lentille présente une distance focale respective qui est différente de la distance focale d'une partie non exposée, et où lesdites parties exposées et non exposées sont situées dans des zones en alternance.

**17.** Procédé selon la revendication 16, comprenant en outre le blocage aux distances focales des parties exposées et non exposées.

**18.** Procédé selon la revendication 16, dans lequel ledit stimulus externe comprend une lumière ultraviolette.

**19.** Procédé selon la revendication 16, dans lequel lesdits changements de propriétés optiques sont provoqués par la polymérisation de la composition modificatrice dans les zones exposées au stimulus externe.

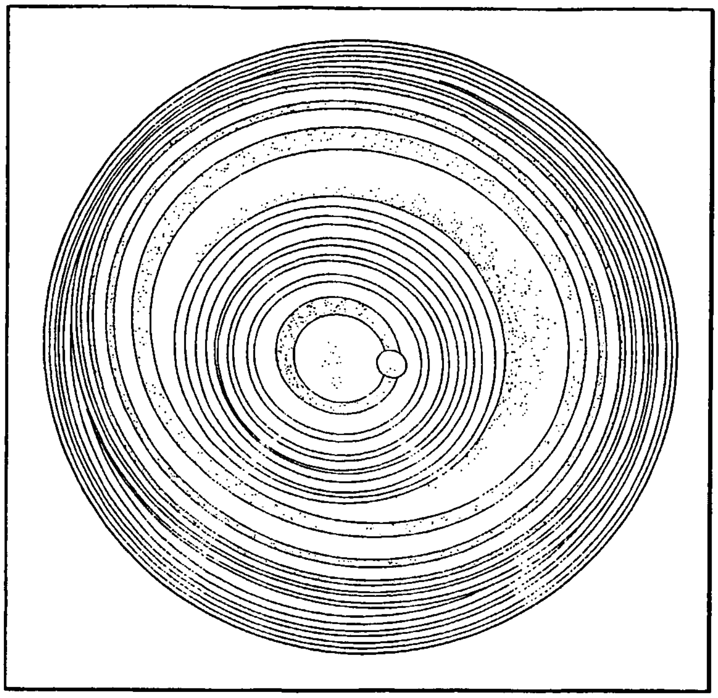

FIG. 1A

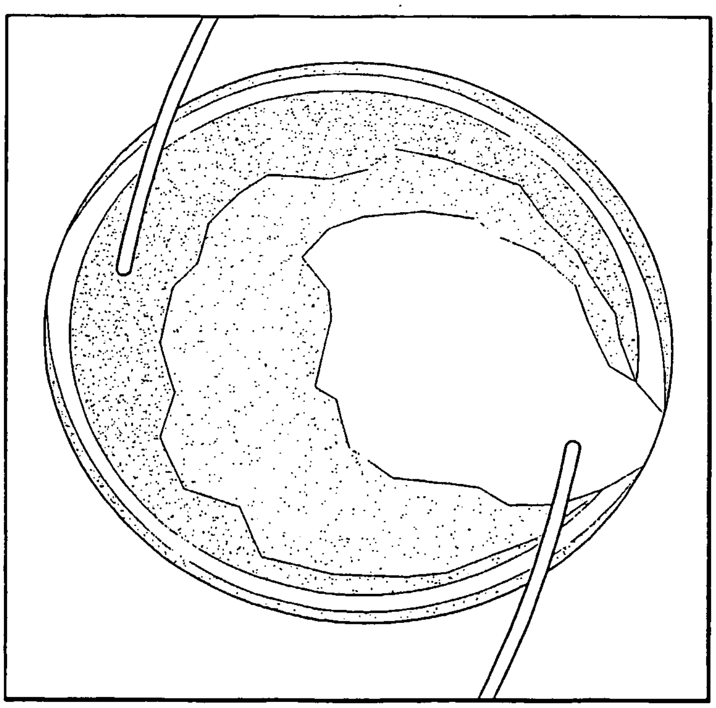

FIG. 1B

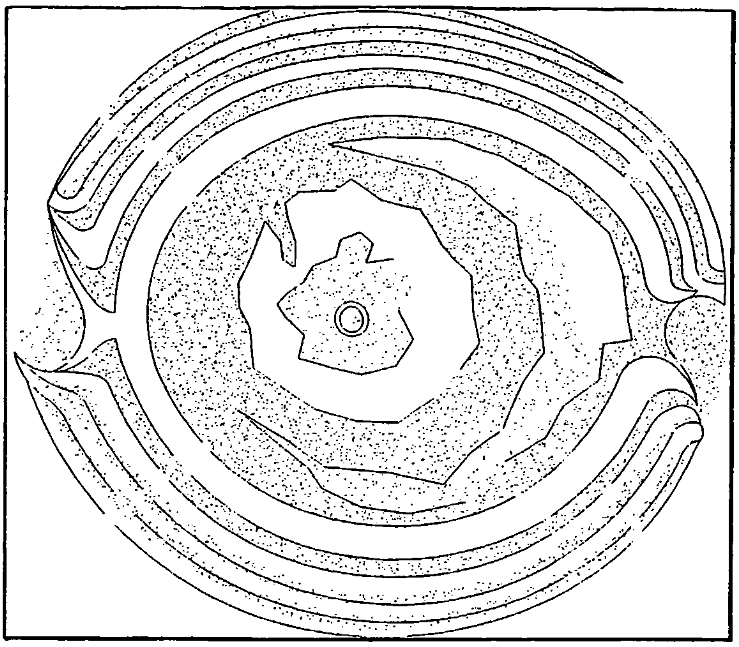

*FIG. 2A*

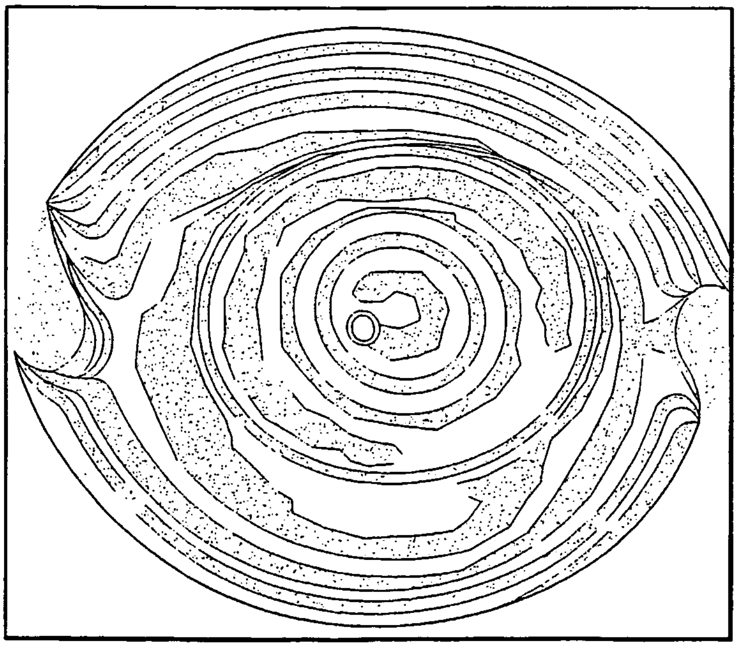

*FIG. 2B*

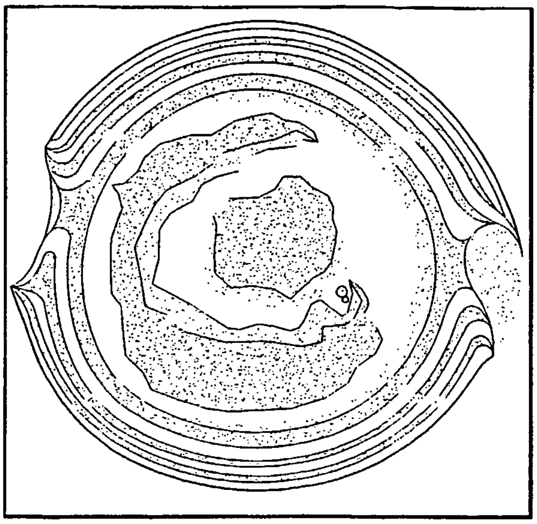

FIG. 3A

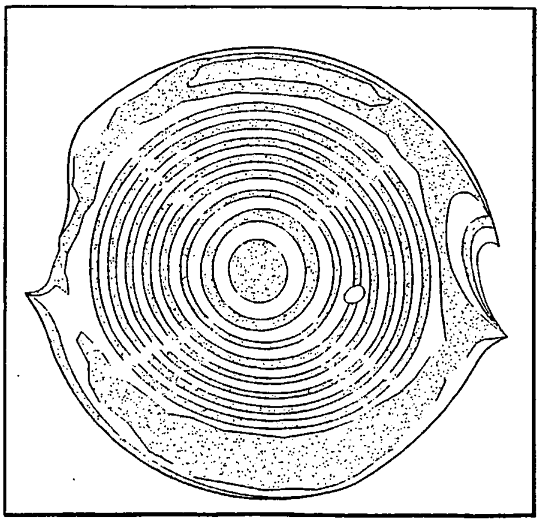

FIG. 3B

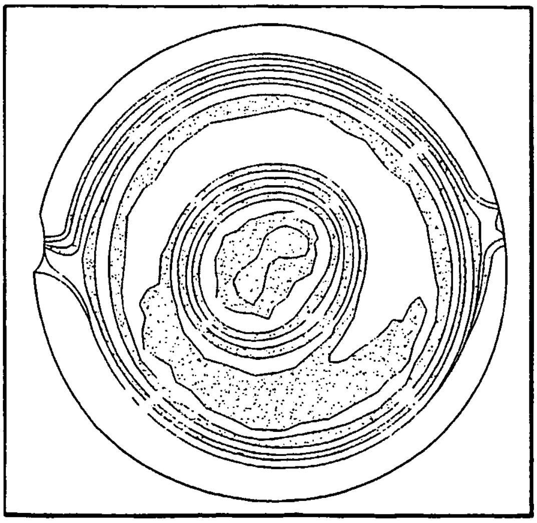

FIG. 3C

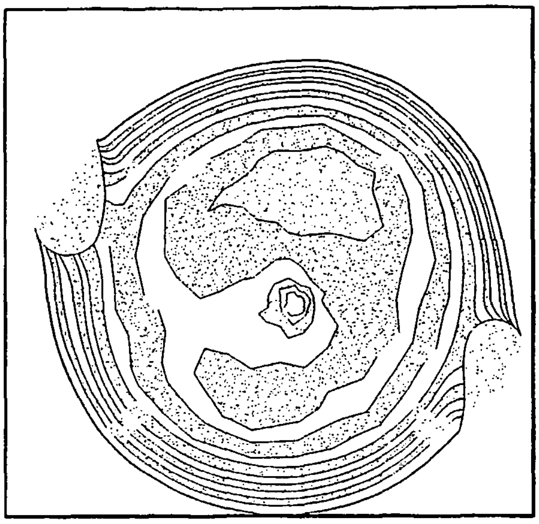

FIG. 4A

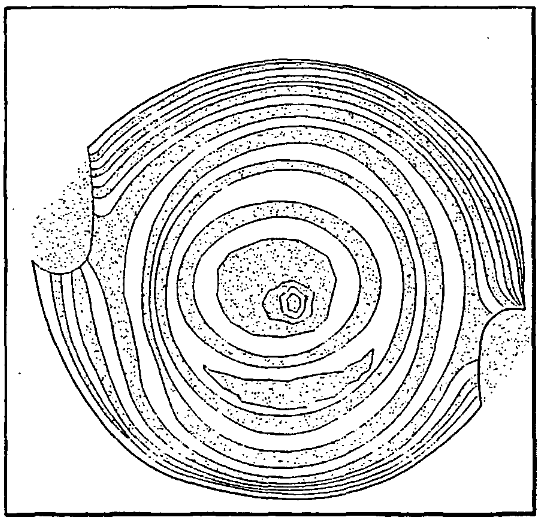

FIG. 4B

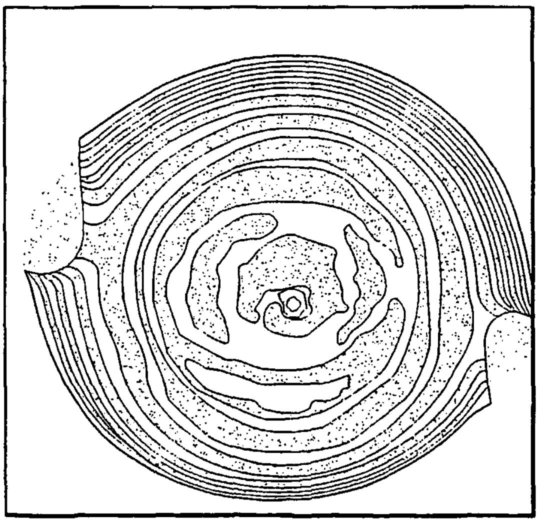

FIG. 4C

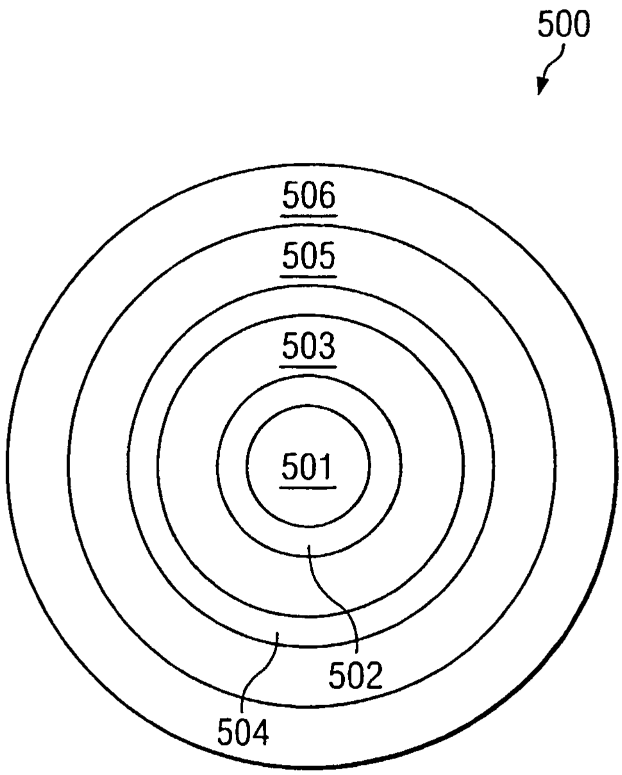


*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5225858 A
- WO 0171411 A
- FR 2657294
- WO 9805272 A
- US 4260725 A
- US 5236970 A
- US 5376694 A
- US 5278258 A
- US 5444106 A


### Non-patent literature cited in the description


- **D. T. Azar.** Intraocular Lenses in Cataract and Refractive Surgery. W. B. Saunders Company, 2001
- **R. L. Stamper ; A Sugar ; D. J. Ripkin.** Intraocular Lenses: Basics and Clinical Applications. American Academy of Ophthalmology, 1993